# EUROPÄISCHE PATENTANMELDUNG

(11) **EP 1 077 048 A2**
(43) Veröffentlichungstag der Anmeldung: **21.02.2001**
(21) Anmeldenummer: 00116853.3
(22) Anmeldetag: 04.08.2000
(51) Int. Cl.: A61B 18/14

(54) **Elektrodenanordnung für medizinische Katheder**

(30) Priorität: 16.08.1999 DE 19938775; 02.11.1999 DE 19952679
(71) Anmelder: Bisping, Hans-Jürgen, Dipl.-Ing., D-52072 Aachen (DE)
(72) Erfinder: Bisping, Hans-Jürgen, Dipl.-Ing., D-52072 Aachen (DE)
(74) Vertreter: Nau, Walter, Dipl.-Ing.

(57) **Zusammenfassung**

Die Erfindung betrifft medizinische Katheter (7), so wie sie z.B. in der Herzschrittmacher- und Defibrillatortherapie sowie bei der elektrophysiologischen Untersuchung verwendet werden. Bekannte Katheter weisen Hülsen (1) bzw. Tipps am distalen Ende auf, deren elektrisch aktive Oberfläche sich zylinderförmig über den gesamten Umfang der Hülse erstreckt.

Davon ausgehend ist die erfindungsgemäße Elektrodenanordnung mit Aussparungen (3) in der Elektrodenhülse (2) versehen und zwar unter Beibehaltung von mind. einem geschlossenen Kreisring (4). Die verbleibende Kontaktfläche (5) ist bei vorgebogenen oder steuerbaren Kathetern an der Außenseite der Katheterkrümmung angeordnet.

## Beschreibung

Die Erfindung betrifft temporäre sowie permanente medizinische Katheter, so wie sie z.B. in der Herzschrittmacher- und Defibrillatortherapie sowie bei der elektrophysiologischen Untersuchung verwendet werden gemäß Oberbegriff von Anspruch 1.

Im letztgenannten Fall kommen diese entweder als diagnostische Katheter mit reiner Detektionsfunktion zum Einsatz, oder als therapeutische Katheter mit Detektions- , Stimulations- und Ablationsfunktion mittels HF-Energie.

Denkbar ist auch eine erfindungsgemäße Anwendung sowohl bei nichtsteuerbaren als auch bei steuerbaren Kathetern.

Die Bauweise derartiger Katheter ist im Prinzip seit Jahrzehnten die gleiche:

Über einen Kunststoffschlauch mit Hohlräumen, der z.B. aus PVC, Nylon, Polyurethan oder ähnlichem Material besteht, werden hülsenförmige Elektroden aus Edelstahl oder Pt/Ir aufgezogen und mit sich im Inneren des Katheters befindlichen Drähten zur Überleitung der elektrischen Energie verbunden.

Eine typische Elektrodengröße ist z.B. 6F (1F entspricht ungefähr 0,33mm) bei einer Hülsen- (Elektroden)länge von 2-10mm. Während die diagnostischen Katheter i.d.R. eine bestimmte vorgegebene Krümmung an ihrem distalen Ende aufweisen, sind die steuerbaren Katheter im Ruhezustand zunächst gerade und können vom distalen Ende durch den Bediener in eine gewünschte Krümmung gebracht werden, die z.T. weit über 90° Winkel bei einem Krümmungsradius von 2-4cm hinausgeht.

Ziel dieser Vorkrümmung, bzw. -biegung ist, einen je nach Verwendungszweck bestimmten Wandkontakt im Herzen oder in einem anderen Körperteil zu erzielen, um die abgeleitete Signalstärke zu verbessern oder aber auch, für den Fall, dass es sich um eine Stimulations- oder Ablationselektrode handelt, durch guten Wandkontakt mit dem Gewebe eine optimale Energieübertragung zu gewährleisten. Speziell bei der Ablation mit HF-Energie kommt den geometrischen Abmessungen der Elektrodenhülsen, bzw. des sog. Elektrodenkopfes, auch Elektrodentip genannt, eine besondere Bedeutung zu. Im folgenden wird die erfindungsgemäße Elektrodenkonfiguration am Beispiel eines Ablationskatheters erläutert; ähnliche Überlegungen gelten aber auch bei anderen Anwendungen.

Wie bereits erwähnt, wird ein Ablationskatheter an einer bestimmten Stelle im Körper durch entsprechende Manipulation, durch Steifigkeit des Katheters selbst oder aber auch durch eine entsprechend gewählte Vorkrümmung oder bei steuerbaren Kathetern durch eine erzielte Krümmung an das Gewebe angedrückt.
Wenn auch Körpergewebe nachgiebig ist, so kommt doch immer nur ein bestimmter Teil der Elektrodenhülse bzw. des Elektrodentipps mit dem Gewebe in innigen Kontakt, während der andere Teil der Elektrode mehr oder weniger ohne Wandkontakt zu liegen kommt und dann zwangsläufig von Gewebeflüssigkeit - bei Ablationskathetern für die kardiologische Applikation also von Blut - umspült ist. Da Blut jedoch einen niedrigeren Widerstand aufweist als Gewebe, wird die abgegebene Energie entsprechend den Widerstandsverhältnissen verteilt. Dies führt dazu, dass umgebenes Blut unerwünscht erhitzt wird, anstatt dass die HF-Energie auf das Gewebe gelenkt wird, um dort die entsprechenden gewünschten Läsionen zu erzielen.

Aus diesem Grunde ist z.B. in der US-Patentschrift 5,643,255 eine Elektrodenkonfiguration vorgeschlagen worden, die an ihrer Katheterspitze eine Tippelektrode trägt, die an einer Seite isoliert ist und drehbar gelagert ist, so dass von außen die Elektrodenlage so eingestellt werden kann, bis die nicht isolierte Fläche mit dem zu abladierenden Gewebe in Kontakt gekommen ist und somit das gewünschte Ablationsergebnis erzielt wird. Teile einer Elektrodenfläche zu isolieren, erfordert jedoch einen relativ aufwendigen Herstellungsprozess.

Auch sind sog. Splitelektroden bekannt, die in der Längsachse eine 2-geteilte Elektrodenhülse bzw. einen 4-fach geteilten Elektrodenkopf aufweisen.

Selbstverständlich müssen die einzelnen Elektrodenelemente in den obengenannten Fällen elektrisch gegeneinander isoliert sein. Schwierig gestaltet sich bei diesen Konzepten die mechanische Anordnung der Elektrodenhülsen auf dem Kunststoffschlauch.

Ziel der Erfindung ist es daher, eine Elektrodenkonfiguration vorzustellen, die einerseits günstige elektrische Eigenschaften garantiert und andererseits leicht herstellbar ist und zuverlässige mechanische Funktion gewährleistet.

Diese Aufgabe wird durch die kennzeichnenden Merkmale des Anspruchs 1 gelöst.

Während bei den bisher bekannten Elektrodenhülsen die gesamte Oberfläche in Wirkverbindung mit den sie umgebenden Medien steht, wird erfindungsgemäß vorgeschlagen, dass die Elektrodenhülse bzw. der Elektrodentipp Aussparungen in beliebiger Größe und Form aufweist. Diese Aussparungen können in Form von einzelnen oder mehreren Bohrungen, Schlitzen, Ausstanzungen oder Ausfräsungen in beliebiger Kurvenform vorliegen. Wichtig ist jedoch, dass eine derartig flächenreduzierte Elektrodenhülse in der bewährten Art und Weise auf dem Kunststoffschlauch befestigt werden kann. Dies wird erfindungsgemäß dadurch gewährleistet, dass die Aussparungen so angeordnet sind, dass zumindest ein i.d.R. kreisförmig den Katheterschlauch umfassender Elektrodenhülsenring bestehen bleibt.

In Bezug auf den sog. Elektrodentipp, also um die am distalen Ende des Katheters angebrachte Elektrode, wird folgendes vorgeschlagen: i.d.R. besteht der Elektrodentipp aus einer Elektrodenhülse, bei der die distale Öffnung durch eine Kappe, die Elektrodenspitze, verschlossen ist.
Die erfindungsgemäße Aussparung wird in diesem Fall so angeordnet, dass die Elektrodentipphülse zumindest einen für die Festigkeit benötigten geschlossenen Elektrodenring aufweist, der z.B. am proximalen Ende angeordnet ist. Um aber auch zu gewährleisten, dass der Elektrodentipp an seinem distalen Ende noch festen Halt auf dem Kunststoffschlauch besitzt, soll erfindungsgemäß die Aussparung so angeordnet sein, dass die Elektrodenkappe über die Mittelachse des Katheterschlauches hinausgeht, so dass ein weiterer distaler geschlossener "Ring" entsteht.

Die durch die oben genannten Aussparungen erzielten Elektrodenkonfigurationen weisen reduzierte Oberflächen auf. Diese sollen vorzugsweise dem zu therapierenden Gewebe zugewandt sein. Es wird daher vorgeschlagen, dass diese 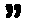 Restflächen" bei vorgebogenen, bzw. von außen biegbaren Kathetern im wesentlichen an der Außenseite der Katheterkrümmung angeordnet sind.

An den Übergängen von den Aussparungen zu den Elektrodenflächen sind nach einer bevorzugten Ausgestaltung der Erfindung im Kunststoffschlauch und /oder unterhalb der Elektrodenhülse Sensoren zur Energiesteuerung /regelung angebracht. Dabei handelt es sich vorzugsweise um Temperatursonden zur Steuerung bzw. Regelung der Kathetertemperatur bei HF-Ablation.

In weiterer Ausgestaltung der Erfindung wird vorgeschlagen, daß anstelle der kreisförmigen Konfiguration des Katheterschlauches sowie der Elektrodenhülse und des Elektrodentipps diese zumindest in Teilbereichen ellipsenförmig ausgestaltet ist. Diese Variante hat den Vorteil, dass die gesamte Katheteranordnung eine biegeweiche und eine biegesteife Krümmungsrichtung hätte; biegeweich in der Richtung, in der der geringere Radius der Katheterkonfiguration angeordnet ist und biegesteif in der Richtung, in der der größere Radius der Katheteranordnung vorliegt.
Für den Anwender hat dieses Konzept den Vorteil, dass er in der biegesteifen Richtung höhere Anpresskräfte ausüben kann. Insofern wird weiterhin vorgeschlagen, dass die Restflächen, die durch eine entsprechende Aussparung übrig bleiben, an einer der ellipsenförmigen Katheterseiten mit dem engeren Krümmungsradius angeordnet sind.

Einfacher in der Herstellung ist es, wenn man die Aussparung so ausgestaltet, dass lediglich ein geschlossener Kreisring und eine Kontaktfläche mit einem freien Ende entsteht.
Zwei oder mehrere derartige Elektrodenhülsen werden dann derart auf einem Katheterschlauch angeordnet, dass das freie Ende einer derartigen Elektrodenhülse unter dem geschlossenen Kreisring der benachbarten Elektrodenhülse zu liegen kommt.
Es wird vorgeschlagen, dass auch mehrere Elektrodenhülsenkonfigurationen derart verschachtelt oder verkettet werden, dass sie z.B. tripolare oder quadripolare Konfigurationen bilden.

In weiterer Ausgestaltung der Erfindung wird vorgeschlagen, dass die gesamte Außen- oder Innenfläche des geschlossenen Kreisringes mit einer Isolierschicht versehen ist. Die auf der Innenseite aufgebrachte Isolierschicht dient dabei zur elektrischen Isolierung gegenüber der Kontaktfläche einer benachbarten Elektrodenhülse; die außen angeordnete Isolierung verhindert, dass sich die Fläche des geschlossenen Kreisringes zur Gesamtkontaktfläche aufaddiert.

Weitere vorteilhafte Ausgestaltungen der Erfindung sind der Zeichnungsbeschreibung zu entnehmen, in der die Ausführungsbeispiele der Erfindung näher beschrieben sind.

Es zeigen:
- Fig. 1:: Eine perspektivische Ansicht einer herkömmlichen Elektrodenhülse,
- Fig. 2:: Eine perspektivische Ansicht einer Elektrodenhülse mit Aussparung,
- Fig. 3:: Die Seitenansicht der Hülse mit Aussparung nach Fig. 2,
- Fig. 4:: Die Seitenansicht einer Hülse mit ellipsenförmiger Aussparung,
- Fig. 5:: Die Seitenansicht einer Hülse mit 2 bogenförmigen Aussparungen,
- Fig. 6:: Die Seitenansicht eines Katheterteils mit 2 darauf angeordneten erfindungsgemäßen Elektrodenhül-sen,
- Fig. 7:: Die Seitenansicht eines distalen Katheterendes mit erfindungsgemäßem Elektrodentipp und Elektrodenhülse,
- Fig. 8:: Die Seitenansicht eines Elektrodentipps,
- Fig. 9:: Eine perspektivische Ansicht einer Elektrodenhülse mit großer Aussparung,
- Fig. 10:: Die Seitenansicht der Hülse nach Fig. 9
- Fig. 11:: Die Seitenansicht von 2 verschachtelten Hülsen nach Fig. 9,
- Fig. 12:: Die Seitenansicht eines Elektrodentipps mit seitlich angebrachten ellipsenförmigen Aussparungen,
- Fig. 13:: Schnittdarstellung des Elektrodentipps nach Fig. 12; Schnitt AB,
- Fig. 14:: Eine perspektivische Ansicht einer Elektrodenhülse mit einer Kontaktfläche mit freiem Ende und einem Kreisring,
- Fig. 15:: Die Seitenansicht der Elektrodenhülse nach Fig. 14,
- Fig. 16:: Die Seitenansicht von 2 verschachtelten Elektrodenhülsen nach Fig. 14.

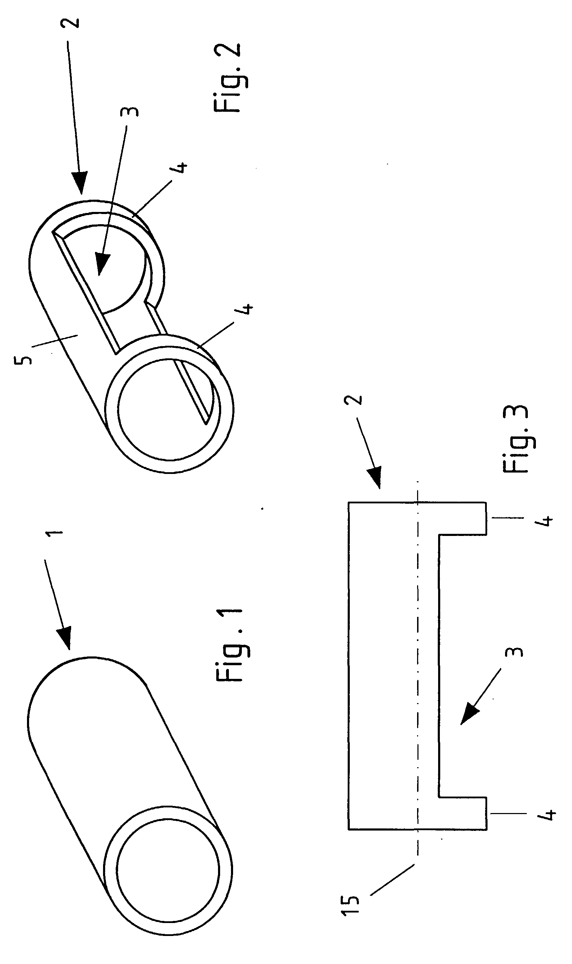

Fig. 1 stellt eine herkömmliche Hülse 1 einer Elektrode mit geschlossener zylindrischer Oberfläche dar. Ihr mechanischer Halt auf dem Katheterschlauch wird durch Verkleben oder Schrumpfungs- bzw. Quellprozesse bewirkt.

In dem Ausführungsbeispiel nach Fig. 2 ist eine erfindungsgemäße Elektrodenhülse 2 mit rechteckförmiger Aussparung 3 dargestellt. Eine elektrische Kontaktfläche 5 wird jetzt im wesentlichen von der Restfläche gebildet, wobei die verbleibenden Kreisringe 4 eine gute mechanische Fixation auf dem Katheterschlauch gewährleisten. Kommt eine derartige Elektrodenhülse 2 mit Aussparung 3 in einer Krümmung eines Katheters 7 zu liegen, so wirkt sich die Aussparung 3 auch dahingehend positiv aus, dass sie die Flexibilität und Biegbarkeit des gesamten Katheters 7 an dieser Stelle erhöht, da sich das Katheterschlauchmaterial in diesem Bereich der Aussparung 3 bei Krümmung 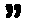 ausbauchen" kann.

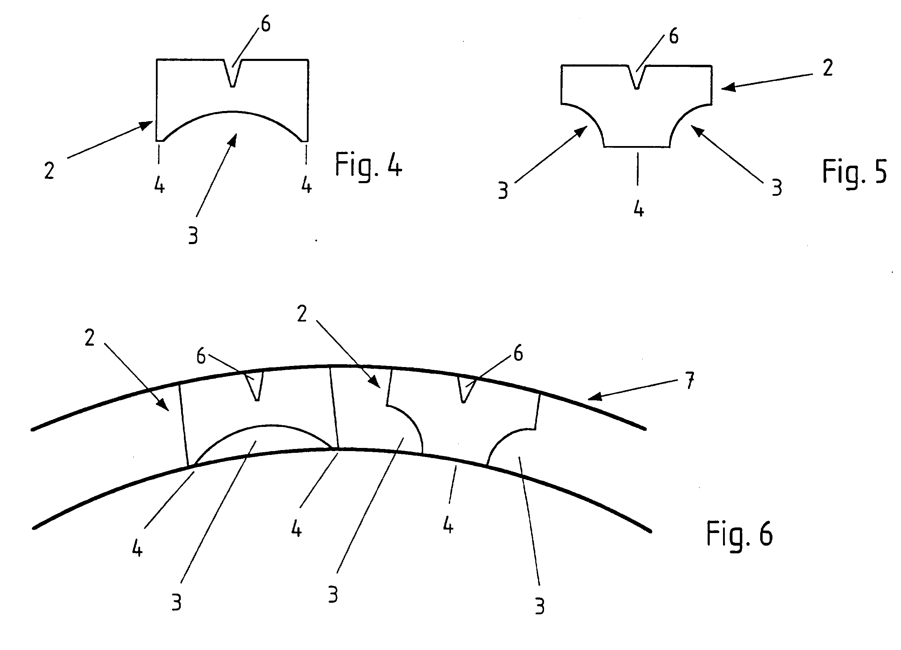

Fig. 3 stellt die Seitenansicht der Elektrodenhülse 2 von Fig. 2 dar, wobei die verbleibenden Kreisringe 4 die mechanische Stabilität garantieren. Ohne diese Kreisringe 4 wäre die reduzierte Kontaktfläche 5 nur mit sehr großem Aufwand auf dem Katheterschlauch zu befestigen. In den Fig. 4 und 5 sind Variationen der Aussparungen 3 aufgezeigt. Man erkennt in Fig. 4 eine ellipsenförmige Aussparung 3, die so angeordnet ist, dass an den beiden Enden der Elektrodenhülse 2 noch geschlossene Kreisringe 4 übrig geblieben sind. Mit 6 ist eine Einkerbung bzw. ein Schlitz bezeichnet, welcher dazu dient, bei Krümmung des Katheters 7 die Flexibilität zu erhöhen. Es sind erfindungsgemäß mehrere derartige Schlitze in der verbleibenden Elektrodenoberfläche denkbar. In dem Ausführungsbeispiel nach Fig. 5 sind die Aussparungen 3 an den beiden Enden der Elektrodenhülse 2 so angeordnet, dass der für die mechanische Festigkeit notwendige geschlossene Kreisring 4 etwa in der Mitte der Elektrodenhülse 2 angeordnet ist.

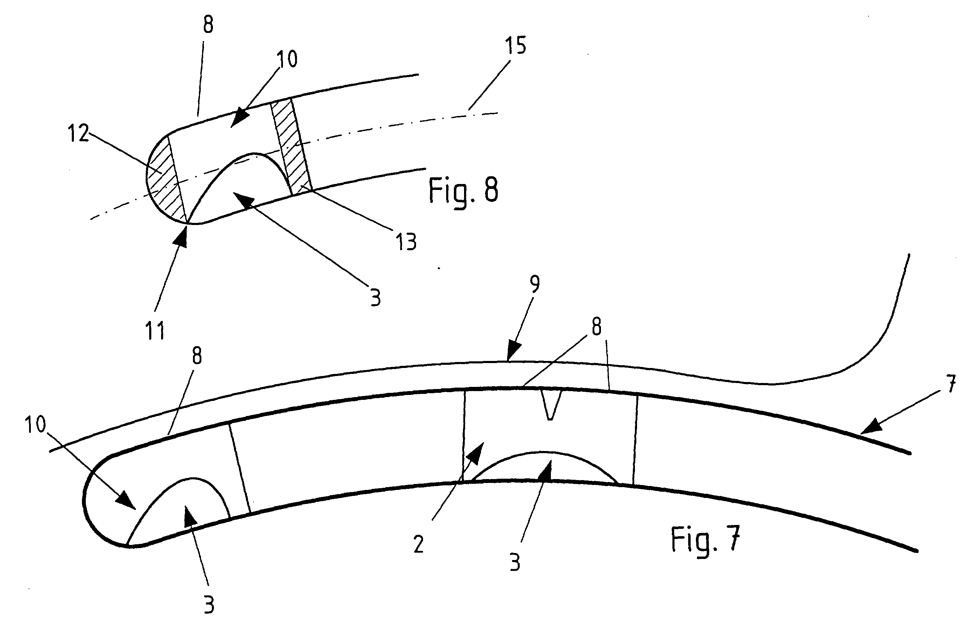

Die Fig. 6 zeigt einen Teil eines Katheters 7 mit Krümmung, auf dem zwei Elektrodenhülsen 2 nach Fig. 4 sowie Fig. 5 aufgebracht sind. Man beachte, dass sich die Aussparungen 3 in beiden Fällen auf der Innenseite des Krümmungsradius befinden und demgemäß die größte elektrisch wirksame Fläche der Elektrodenhülsen 2 auf der Außenseite der Krümmung des Katheters 7 zu liegen kommt.

In Fig. 7 wird aufgezeigt, wie sich der Katheter 7 mit einem Elektrodentipp 10 und einer Elektrodenhülse 2 mit Abstand vom Elektrodentipp 10 mit seiner Außenfläche an einer Gewebewand 9 abstützt. Der Elektrodentipp 10 wie auch die Elektrodenhülse 2 sind auch hier wiederum derart angeordnet, dass ihr größter Flächenbereich 8 auf der Außenseite der Katheterkrümmung zu liegen kommt, so dass zu erwarten ist, dass sie in gutem elektrischen Wirkkontakt mit der Gewebewand 9 stehen.

In Fig. 8 wird eine vorteilhafte Ausgestaltung der Elektrodenspitze bzw. des Elektrodentipps 10 aufgezeigt. Die Aussparung 3 ist dabei so angeordnet, dass am proximalen Ende des Elektrodentipps ein kreisförmiger Ring 13 - schraffiert dargestellt - verbleibt. Seine Existenz garantiert dafür, dass der Elektrodentipp 10 einen festen Halt auf dem Katheterschlauch erfährt. Die distale Schnittlinie 11 der Aussparung 3 mit einer Elektrodenkappe 12 ist gemäß Fig. 8 so gewählt, dass die Elektrodenkappe 12 noch groß genug ist, um noch einen für die mechanische Festigkeit erforderlichen geschlossenen Ring - schraffiert dargestellt - , zu bilden. Das ist immer dann der Fall, wenn die Elektrodenkappe 12 über die Mittelachse 15 des Katheterschlauches hinausgeht.
Bei Vergrößerung der Aussparung 3 in Richtung auf das distale Ende des Katheters 7 würde die Schnittlinie 11 weiter zur Mittelachse 15 des Katheters hin wandern bis schließlich kein geschlossener 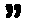 Ersatzring" mehr gebildet ist. Dann wäre die mechanische Fixation des gesamten Elektrodentipp 10 in Frage gestellt.

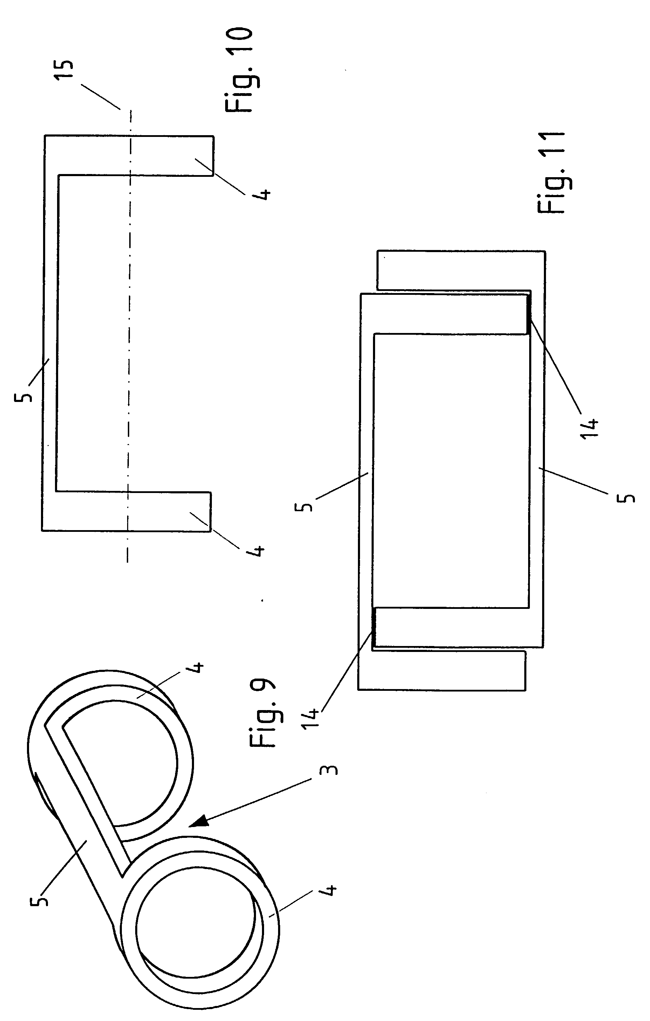

In den Fig. 9 - 11 sind weitere Variation der Aussparung 3 aufgezeigt. Hierbei handelt es sich um eine extrem große Aussparung 3, die einerseits noch zwei geschlossene Kreisringe 4 verbleiben lässt, andererseits aber eine sehr schmale Kontaktfläche 5 übrig lässt. Es wird vorgeschlagen, dass die durch die Aussparung 3 reduzierte Oberfläche mindestens 70% der gesamten äußeren Oberfläche der Elektrodenhülse 2 beträgt, also die verbleibende Kontaktfläche 5 höchstens 30% beträgt. Verschachtelt man zwei derartige ausgesparte Elektrodenhülsen nach Fig. 5 derart, wie es in Fig.11 dargestellt ist und verhindert man den elektrischen Kontakt zwischen beiden Elektrodenhülsen durch Isolierschichten 14, so liegen zwei elektrisch voneinander getrennte Kontaktflächen 5 gegenüber und man hat es mit einem orthogonalen Elektrodensystem zu tun, welches bekannte Vorteile bei der Signalableitung aufweist. Der Vorzug dieser Anordnung liegt darin, dass sie relativ einfach herzustellen ist und gleichzeitig hohe mechanische Festigkeit aufweist. Es wird vorgeschlagen, dass auch andere erfindungsgemäße Elektrodenhülsenkonfigurationen derart verschachtelt werden.

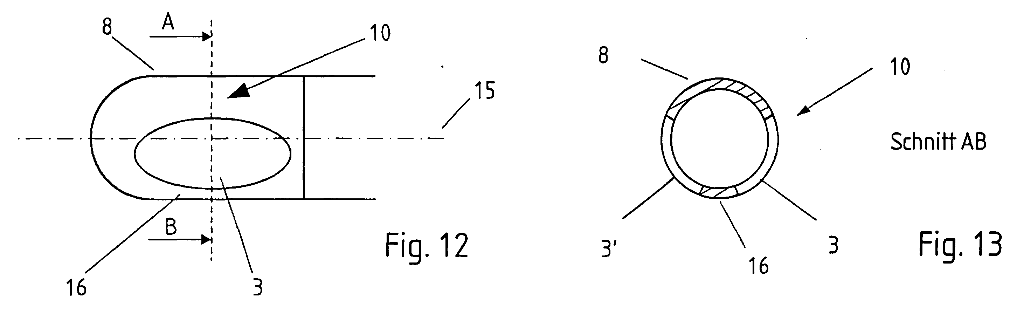

Das Ausführungsbeispiel nach Fig. 12 weist eine weitere Variation der Aussparung 3 auf. Hierbei sind am Elektrodentipp 10 zwei im wesentlichen seitlich angeordnete Aussparungen 3, 3*'* angeordnet, wobei in der Fig. 12 allerdings nur eine zu erkennen ist, während die andere symmetrisch dazu auf der anderen Seite zu liegen kommt. Bei dieser dargestellten Modifikation ist der am proximalen Teil schon aus der Fig. 8 bekannte geschlossene Ring 13 vorhanden. Zusätzlich verbleibt ein geschlossener Steg 16 auf der in der Fig. 12 gezeigten Elektrodentippunterseite, der wiederum die gewünschte mechanische Stabilität gewährleistet. In Fig. 13 ist die Schnittdarstellung der vorhergenannten Anordnung mit Schnitt bei AB gezeigt.

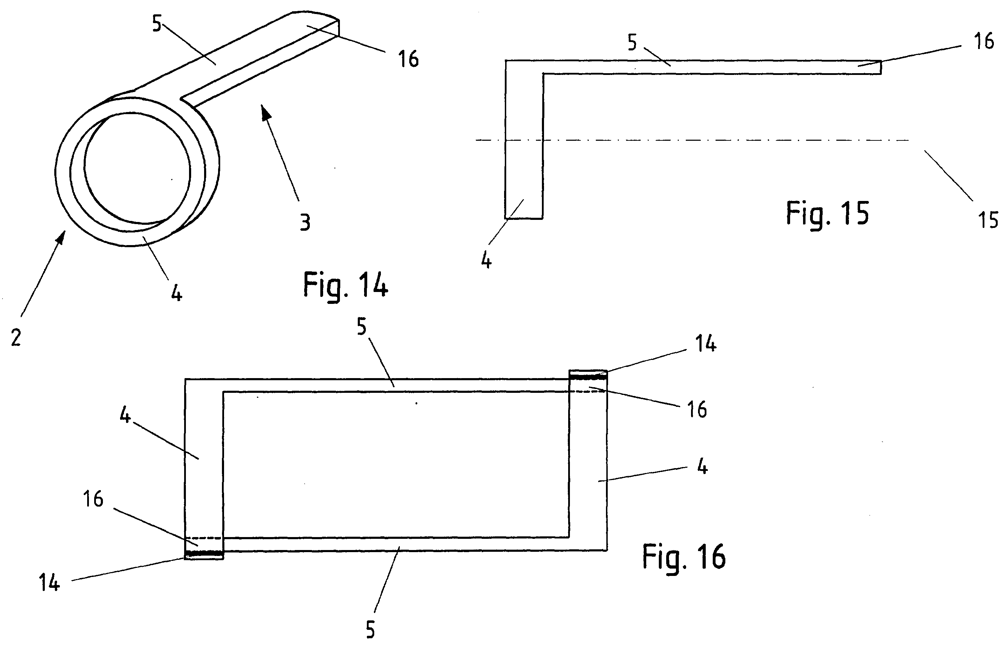

In dem Ausführungsbeispiel nach Fig. 14 ist eine Elektrodenhülse 2 mit rechteckförmiger Aussparung 3 dargestellt. Die elektrische Kontaktfläche 5 wird jetzt im wesentlichen von der Restfläche gebildet. Ein Ende der Kontaktfläche 5 geht in einen Kreisring 4 über, der eine gute mechanische Fixation auf dem Katheterschlauch gewährleist. Das zweite Ende der Kontaktfläche 5 endet in einem freien Ende 16.

Fig. 15 stellt die Seitenansicht der Elektrodenhülse von Fig. 14 dar.

Verschachtelt man zwei Elektrodenhülsen 2 nach Fig. 14 derart, wie es in Fig. 16 dargestellt ist und verhindert man den elektrischen Kontakt zwischen beiden Elektrodenhülsen 2 durch Isolierschichten 14, so liegen zwei elektrisch voneinander getrennte Kontaktflächen 5 gegenüber und man hat es mit einem orthogonalen Elektrodensystem zu tun, welches bekannte Vorteile bei der Signalableitung aufweist. Der Vorzug dieser Anordnung liegt darin, dass sie einfach herzustellen ist und trotzdem hohe mechanische Festigkeit aufweist.

## Patentansprüche

1. Temporäre oder permanente medizinische Katheter (7) für die Ableitung von Körpersignalen bzw. für die Überleitung von Energie auf Körperteile mit Elektrodenelementen aus beidseitig offenen Hülsen (1) oder aus einseitig offenen Hülsen mit die andere Öffnung abdeckenden Kappen,
**dadurch gekennzeichnet, dass** die Elektrodenelemente mindestens eine oberflächenreduzierende Aussparung (3) aufweisen.

2. Medizinischer Katheter (7) nach Anspruch 1,
**dadurch gekennzeichnet, dass** das Elektrodenelement mindestens einen geschlossenen Kreisring (4) oder Ring (13) aufweist.

3. Medizinischer Katheter (7) nach einem der vorhergehenden Ansprüche,
**dadurch gekennzeichnet, dass** die Aussparungen (3) in beliebiger Form und an beliebiger Stelle in einer Elektrodenhülse (2) angeordnet sind.

4. Medizinischer Katheter (7) nach einem der vorhergehenden Ansprüche,
**dadurch gekennzeichnet, dass** die nach Anbringung der Aussparungen (3) verbleibenden Kontaktflächen (5) und Flächenbereiche (8) im wesentlichen an der Außenseite einer eventuell vorhandenen Katheterkrümmung angeordnet sind.

5. Medizinischer Katheter (7) nach einem der vorhergehenden Ansprüche,
**dadurch gekennzeichnet, dass** Elemente zur Begrenzung der Energieabgabe in Form von z.B. Thermistor oder Thermocouple so angeordnet sind, daß sie in der Nähe der Übergänge zwischen den Aussparungen (3) der Kontaktflächen (5) oder Flächenbereiche (8) zu liegen kommen.

6. Medizinischer Katheter nach einem der vorhergehenden Ansprüche,
**dadurch gekennzeichnet, dass** ein Elektrodentipp (10) mindestens einen geschlossenen Ring (13) aufweist.

7. Medizinischer Katheter (7) nach einem der vorhergehenden Ansprüche,
**dadurch gekennzeichnet, dass** eine Elektrodentippkappe (12) sich über die Mittelachse (15) des Katheterschlauches erstreckt und somit wiederum einen geschlossenen Ring bildet.

8. Medizinischer Katheter (7) nach einem der vorhergehenden Ansprüche,
**dadurch gekennzeichnet, dass** zwei Elektrodenhülsen (2) mit Aussparungen (3) derart auf einem Katheterschlauch angeordnet werden, dass die außenliegenden Kontaktflächen (5) diagonal gegenüber angeordnet sind und Isolierelemente (14) vorhanden sind, die einen elektrischen Kontakt zwischen beiden Elektrodenhülsen (2) verhindern.

9. Medizinischer Katheter (7) nach einem der vorhergehenden Ansprüche,
**dadurch gekennzeichnet, dass** die Elektrodenhülsen (2) an ihren Enden jeweils eine Aussparung (3) aufweisen und dass zwischen den Aussparungen (3) ein geschlossener Kreisring (4) angeordnet ist.

10. Medizinischer Katheter nach einem der vorhergehenden Ansprüche,
**dadurch gekennzeichnet, dass** am Elektrodentipp (10) zwei Aussparungen (3, 3') und ein zwischen den Aussparungen verbleibender Steg (16) angeordnet sind.

11. Medizinischer Katheter (7) nach einem der vorhergehenden Ansprüche,
**dadurch gekennzeichnet, dass** der Katheter (7) oder Teilbereiche des Katheters sowie die Elektrodenhülse (2) und der Elektrodentipp (10) ellipsenförmig ausgestaltet sind.

12. Medizinischer Katheter nach einem der vorhergehenden Ansprüche,
**dadurch gekennzeichnet, dass** die nach Anbringung der Aussparungen (3) verbleibenden Kontaktflächen (5) oder Flächenbereiche (8) im wesentlichen an dem Teil der ellipsenförmigen Seite des Katheters (7) angeordnet sind, der den geringeren Krümmungsradius aufweist.

13. Medizinischer Katheter (7) nach einem der vorhergehenden Ansprüche,
**dadurch gekennzeichnet, dass** die Elektrodenhülse (2) einen geschlossenen Kreisring (4) und eine Kontaktfläche (5) mit einem freien Ende (16) aufweist, dass mindestens zwei Elektrodenhülsen auf einem gemeinsamen Katheterschlauch angeordnet sind und dass ihre freien Enden (16) jeweils unter den Kreisring (4) einer benachbarten Elektrodenhülse (2) geschoben sind.

14. Medizinischer Katheter (7) nach einem der vorhergehenden Ansprüche,
**dadurch gekennzeichnet, dass** die Kontaktflächen (5) unter beliebigen Winkeln zueinander verdreht angeordnet sind.

15. Medizinischer Katheter (7) nach einem der vorhergehenden Ansprüche,
**dadurch gekennzeichnet, dass** die Innen- und/oder Außenseiten der Kreisringe (4) ganz oder teilweise mit einer elektrischen Isolierschicht umgeben sind.
